# EUROPEAN PATENT APPLICATION

(11) **EP 3 127 571 A1**
(43) Date of publication of application: **08.02.2017**
(21) Application number: 15773011.0
(22) Date of filing: 26.02.2015
(51) Int. Cl.: A61M 5/315, A61M 5/28

(54) **SYRINGE GASKET AND SYRINGE EQUIPPED WITH SAID GASKET**

(30) Priority: 31.03.2014 JP 2014072138
(71) Applicant: Terumo Kabushiki Kaisha, Tokyo 151-0072 (JP)
(72) Inventor: ABE, Yoshihiko, Ashigarakami-gun Kanagawa 259-0151 (JP); UEDA, Tsutomu, Fujinomiya-shi Shizuoka 418-0004 (JP)
(74) Representative: Casalonga
(86) International application number: PCT/JP2015/055643
(87) International publication number: WO 2015/151671

(57) **Abstract**

A gasket (1) for use in a syringe has a core part (2) and a coating layer (3) formed on the core part (2) at least a portion thereof which contacts the syringe. In a portion where the coating layer is formed, an outer surface of the core part (2) is not exposed, and an outer surface layer of the core part (2) is formed of the coating layer (3). The coating layer (3) is an elastic solidified material layer made of a silicone-based resin composition. In an enlarged image of the coating layer (3) observed in an axial section of the gasket by using a transmission electron microscope, the coating layer has island-shaped solidified parts and sea-like solidified parts each positioned between the island-shaped solidified parts and linking the island-shaped solidified parts to each other and does not substantially have pin holes. The island-shaped solidified part is composed mainly of aggregates of reactive silicone.

## Description

### TECHNICAL FIELD

The present invention relates to a gasket for use in a syringe having a stable sliding contact performance and the syringe having the gasket.

### BACKGROUND ART

A prefilled syringe in which a liquid medicine is filled in advance has been conventionally used to prevent use of a wrong medicine, prevent hospital infection, disposability, and increase efficiency in hospital service. A syringe including a syringe to be used as the prefilled syringe is constructed of an outer cylinder, a gasket slidable inside the syringe, and a plunger for operating the movement of the gasket. To enhance the sliding contact performance of the gasket and obtain a high degree of flow accuracy without generating a large irregularity in the discharge of the liquid medicine, silicone oil or the like is applied to the inner surface of a sliding contact portion of the outer surface of the gasket or the inner surface of the syringe as a lubricant. But it is known that in dependence on a liquid medicine, an interaction occurs between the liquid medicine and the lubricant such as the silicone oil. When the liquid medicine is stored for a long time after the liquid medicine is filled in the syringe, the liquid medicine is modified by the interaction. Thus it is difficult to use some kinds of medicines for the prefilled syringe.

The prefilled syringe to be stored for a long time with the liquid medicine filled therein is demanded to keep the liquid medicine stable and eliminate the need for the lubricant.

To solve the above-described problem, as disclosed in a patent document 1 (Japanese Patent Application Laid-Open Publication No. 62-32970) and in a patent document 2 (Japanese Patent Application Laid-Open Publication No. 2002-089717, U.S. Patent No. 7111848), prefilled syringes were proposed in which the surface of the gasket is covered with the fluorine resin which is a material having a lower friction coefficient than the material of the core part to eliminate the use of the lubricant.

The present applicant proposed the gasket formed liquid-tightly and slidably inside the plastic outer cylinder of the syringe, as disclosed in the Japanese Patent No. 5394256 (patent document 3). The proposed gasket has the core part made of the elastic body and the coating layer formed at least the portion thereof which contacts the syringe. The coating layer consists of the composition containing the silicone-based resin made of the condensate of the reactive silicone having the silanol group at its both terminals and having the siloxane bond derived from the silanol group and does not contain solid microparticles.

### PRIOR ART DOCUMENTS

### PATENT DOCUMENTS

Patent document 1: Japanese Patent Application Laid-Open Publication No. 62-32970
Patent document 2: Japanese Patent Application Laid-Open Publication No. 2002-089717, U.S. Patent No. 7111848
Patent document 3: Japanese Patent No. 5394256

### SUMMARY OF THE INVENTION

### PROBLEM TO BE SOLVED BY THE INVENTION

The gaskets disclosed in the patent document 1 (Japanese Patent Application Laid-Open Publication No. 62-32970) and the patent document 2 (Japanese Patent Application Laid-Open Publication No. 2002-089717, U.S. Patent No. 7111848) can be expected to be effective in dependence on a use condition. But in a preparation for a prefilled syringe demanded to discharge the liquid medicine under a high pressure and have the performance of stably discharging the liquid medicine little by little with very high accuracy for a long time by using a syringe pump or the like, liquid-tightness and sliding contact performance which are fundamental performance demanded for the syringe are still in a trade-off relationship. A syringe which allows these performances to be compatible with each other at a high level and has higher performance is needed.

That is, in administration of the liquid medicine by using the syringe pump, when the liquid medicine is discharged in a condition where the flow rate is so low (for example, in the case of a syringe having a diameter of 24mm, a moving speed is approximately 2mm/hour when a liquid medicine is discharged 1mL/hour therefrom) that the flow of the liquid medicine is invisible, an unstable discharge state called pulsation is liable to occur. Thus there is a fear that accurate administration of the liquid medicine is prevented.

The gasket disclosed in the patent document 3 is liquid-tight and has stable sliding contact performance without applying a lubricant to the sliding contact surface thereof. As a result of investigations conducted by the present inventors, they have found that it is desirable for the coating layer to have a higher strength.

The present invention has been made to solve the above-described problem. It is an object of the present invention to provide a gasket whose sliding contact surface has stable sliding contact performance without applying a lubricant thereto and whose coating layer for imparting sliding contact performance to the gasket hardly gives rise to interlayer fracture and a syringe having the gasket.

### MEANS FOR SOLVING THE PROBLEM

Means for achieving the above-described object is as follows:

A gasket of the present invention for use in a syringe is liquid-tightly slidable inside an outer cylinder of the syringe. The gasket has a core part and a coating layer formed on the core part at least a portion thereof which contacts the syringe. In a portion where the coating layer is formed, an outer surface of the core part is not exposed, and an outer surface layer of the core part is formed of the coating layer. The coating layer is an elastic solidified material layer made of a silicone-based resin composition. In an enlarged image of the coating layer observed in an axial section of the gasket by using a transmission electron microscope, the coating layer has island-shaped solidified parts and sea-like solidified parts each positioned between the island-shaped solidified parts and linking the island-shaped solidified parts to each other and does not substantially have pin holes. The island-shaped solidified part is composed mainly of aggregates of reactive silicone.

Other means for achieving the above-described object is as follows:

A syringe of the present invention has the gasket slidably accommodated inside the outer cylinder and a plunger which is mounted on the gasket or can be mounted thereon.

### BRIEF DESCRIPTION OF THE DRAWINGS

Fig. 1 is a front view showing a gasket of an embodiment of the present invention.
Fig. 2 is a sectional view of the gasket shown in Fig. 1.
Fig. 3 is a plan view of the gasket shown in Fig. 1.
Fig. 4 is a bottom view of the gasket shown in Fig.1.
Fig. 5 is a sectional view of a prefilled syringe in which the gasket shown in Fig. 1 is used.
Fig. 6 shows an enlarged image of a coating layer of the gasket of an embodiment of the present invention observed in an axial section of the gasket by using a transmission electron microscope.
Fig. 7 shows an enlarged image of the coating layer of the gasket of the embodiment of the present invention observed in a direction vertical to the axial section of the gasket by using the transmission electron microscope.
Fig. 8 shows an enlarged image of a coating layer of a gasket of another embodiment of the present invention observed in an axial section of the gasket by using the transmission electron microscope.
Fig. 9 shows an enlarged image of a coating layer of a gasket of still another embodiment of the present invention observed in an axial section of the gasket by using the transmission electron microscope.
Fig. 10 shows an enlarged image of a coating layer of a gasket of a different embodiment of the present invention observed in an axial section of the gasket by using the transmission electron microscope.
Fig. 11 shows an enlarged image of a coating layer of a gasket of a comparison example observed in an axial section of the gasket by using the transmission electron microscope.

### MODE FOR CARRYING OUT THE INVENTION

A gasket of an embodiment of the present invention is described below.

A gasket 1 of the present invention slidably contacts an outer cylinder of a syringe. The gasket 1 has a core part 2 made of an elastic body and a coating layer 3 formed on the core part 2 at least a portion thereof which contacts the syringe. A portion where the coating layer is formed does not expose an outer surface of the core part 2 and forms an outer surface layer of the core part of the coating layer 3. The coating layer 3 is an elastic solidified material layer made of a silicone-based resin composition. In an enlarged image of the coating layer 3 observed in an axial section of the gasket 1 by using a transmission electron microscope, the coating layer 3 has island-shaped solidified parts (island phase solidified parts) and sea-like solidified parts (sea phase solidified parts) each positioned between the island-shaped solidified parts and linking the island-shaped solidified parts to each other. The coating layer does not substantially have pin holes. The island-shaped solidified part is composed mainly of aggregates of reactive silicone.

The gasket of the present invention is described below by using an embodiment in which the gasket is used for a syringe and applied to the syringe.

The gasket 1 of this embodiment is used for the syringe and liquid-tightly and slidably accommodated inside the outer cylinder 11 for the syringe. The gasket 1 has the coating layer 3 formed at a portion thereof which contacts the outer cylinder 11. The coating layer 3 has a microstructure.

The gasket 1 has the core part 2 and the coating layer 3 formed on an outer surface of the core part 2 at least a portion thereof which contacts an inner surface of the outer cylinder. The coating layer 3 may be formed on the entire outer surface of the core part 2.

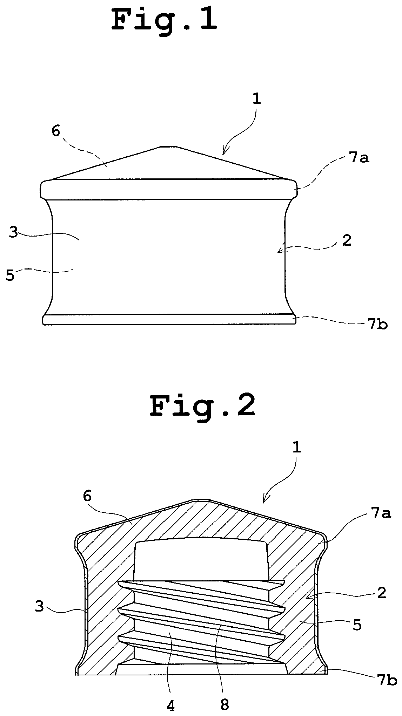

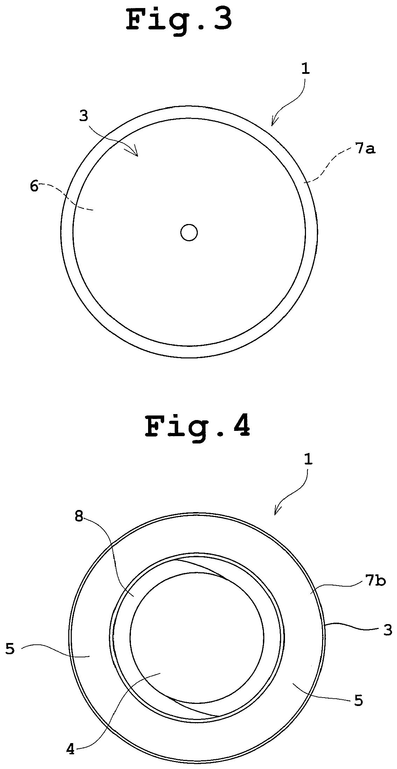

As shown in Figs. 1, 2, and 5, the core part 2 of the gasket 1 for use in the syringe has a main body 5 extending in an almost equal diameter; a tapered portion 6, disposed at a distal side of the main body 5, whose diameter decreases taperingly toward the distal end thereof; a plunger-mounting portion 4 formed inside the main body 5 from a proximal end thereof toward a distal side thereof; a distal-side annular rib 7a formed on a side surface of the distal portion of the main body 5; and a proximal-side annular rib 7b formed on a side surface of the proximal portion of the main body 5. As shown in Figs. 2 and 4, the plunger-mounting portion 4 is formed as an approximately columnar concave portion which extends from the proximal end of the main body 5 to a position in the vicinity of the distal end thereof inside the main body 5. A threaded engaging portion 8 capable of threadedly engaging a threaded engaging portion formed at a distal end of a plunger is formed on a side surface of the concave portion. A distal-end surface of the concave portion is formed almost flatly. The plunger-mounting portion does not necessarily have to be formed as the threaded engaging portion, but may be formed as an engaging portion which engages the distal portion of the plunger.

The outer diameters of the annular ribs 7a and 7b are formed a little larger than the inner diameter of the outer cylinder 11 for use in the syringe. Therefore the annular ribs 7a and 7b compressively deform inside the outer cylinder 11. Although two annular ribs are formed in this embodiment, one or three or more annular ribs may be formed.

As a material for forming the core part 2, an elastic material is preferable. The elastic material is not limited to a specific one, but rubber materials (vulcanized rubber materials) such as natural rubber, isoprene rubber, butyl rubber, chloroprene rubber, nitrile-butadiene rubber, styrene-butadiene rubber, and silicone rubber; styrene elastomer and hydrogenated styrene elastomer; and mixtures of the styrene elastomer and polyolefins such as polyethylene, polypropylene, polybutene, and α-olefin copolymers; mixtures of the styrene elastomer and oil such as liquid paraffin, process oil; and mixtures of the styrene elastomer and powdery inorganic substances such as talc, cast, mica, and the like are listed. Further it is possible to use a polyvinyl chloride elastomer, an olefin elastomer, a polyester elastomer, a polyamide elastomer, a polyurethane elastomer, and mixtures of these elastomers as materials composing the core part 2. As the composing material, the diene rubbers and the styrene elastomer are preferable because these rubbers and elastomers have elastic properties and can be sterilized by y ray, electron beams, and high-pressure steam.

In a case where the core member has the annular ribs slidable with the annular ribs liquid-tightly contacting the outer cylinder, it is necessary to form the coating layer 3 on at least the annular ribs. More specifically, it is necessary that the coating layer 3 is formed on the distal-side annular rib 7a and the proximal-side annular rib 7b. The thickness of the coating layer 3 is 2 to 30µm and favorably to 4 to 12µm. When the thickness of the coating layer 3 is not less than 1µm, the coating layer 3 displays a necessary slidable performance. When the thickness of the coating layer 3 is not more than 30µm, the coating layer 3 does not adversely affect the elasticity of the gasket. It is possible to use solvent-based silicone-based resin dissolved in an organic solvent and water-based silicone-based resin emulsified and dispersed in water. From the viewpoint of the influence on the material of the gasket or the aptitude as a liquid medicine accommodation container, the water-based silicone-based resin is preferable. The coating layer 3 is constructed of a material capable of allowing its friction coefficient to be lower than that of the material for forming the gasket made of only the core part 1.

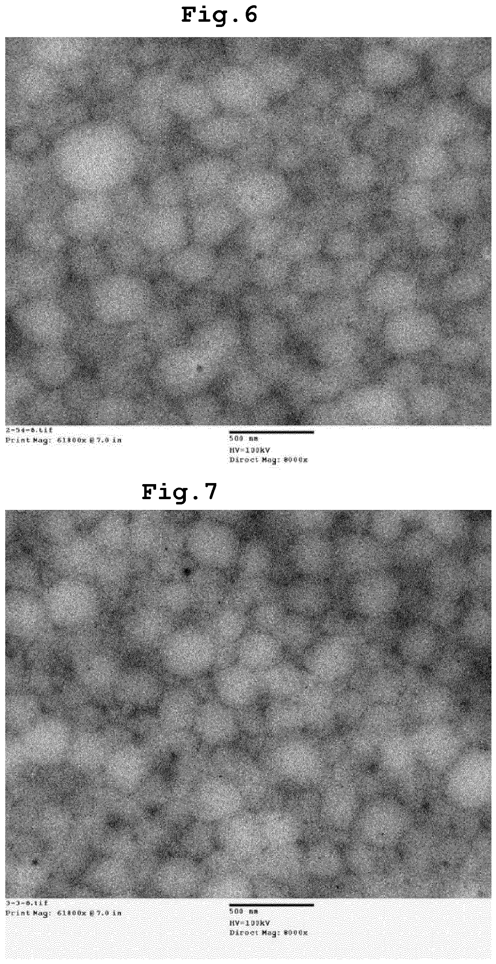

The coating layer 3 is an elastic solidified material layer made of a silicone-based resin composition. As shown in the enlarged image shown in Fig. 6 observed by using the transmission electron microscope, in the enlarged image of the coating layer 3 observed in the axial section of the gasket 1 by using the transmission electron microscope, the coating layer 3 has the island-shaped solidified parts and the sea-like solidified parts each positioned between the island-shaped solidified parts and linking the island-shaped solidified parts to each other. The coating layer does not substantially have pin holes.

As shown in an enlarged image shown in Fig. 7 observed by using the transmission electron microscope, in the enlarged image of the coating layer 3 observed in a section vertical to the axial section of the gasket by using the transmission electron microscope, the coating layer has also the island-shaped solidified parts and the sea-like solidified parts each positioned between the island-shaped solidified parts and linking the island-shaped solidified parts to each other. The coating layer does not substantially have pin holes.

That is, the coating layer 3 has a three-dimensional construction formed of the island-shaped solidified parts and the sea-like solidified parts each positioned between the island-shaped solidified parts and linking the island-shaped solidified parts to each other.

Each island-shaped solidified part is composed mainly of the aggregates of the reactive silicone. Therefore the island-shaped solidified part is sufficiently elastic. The island-shaped solidified part is almost circular. The diameter of the island-shaped solidified part is favorably 0.05 to 0.8µm and especially favorably 0.1 to 0.6µm. The area of each island-shaped solidified part is favorably 0.002 to 0.5µm² and especially favorably 0.007 to 0.3µm². The area occupancy ratio of the entire island-shaped solidified part observed in a section of the coating layer 3 is favorably 80 to 95% and especially favorably 85 to 90%. By setting the occupancy ratio of the entire island-shaped solidified part to the above-described range, the island-shaped solidified parts constitute a main part of the coating layer and allow the coating layer 3 to be sufficiently elastic.

It is preferable that the island-shaped solidified part and the sea-like solidified part have different properties. More specifically, it is preferable that the island-shaped solidified part has a higher elasticity than the sea-like solidified part and that the sea-like solidified part has a higher strength than the island-shaped solidified part. Conversely the sea-like solidified part may have a higher elasticity than the island-shaped solidified part and that the island-shaped solidified part may have a higher strength than the sea-like solidified part. As described above, by differentiating the property of the island-shaped solidified part and that of the sea-like solidified part from each other, it is possible to decrease the possibility of the occurrence of breakage inside the coating layer 3 and the occurrence of delamination.

It is preferable that the coating layer 3 is a compressed solidified material formed due to drying. The compressed solidified material formed due to drying means a solidified material formed owing to a decrease in the thickness of a liquid material caused by volatility of a solvent resulting from drying after the liquid material for forming the coating layer 3 is applied to the core part 2. By forming the coating layer 3 as the drying-caused compressed solidified material, pin holes are not formed in the coating layer 3 and contents of the liquid material for forming the coating layer aggregate. Thus the coating layer has a high adhesiveness to the core part 2 and a high strength.

It is preferable that the sea-like solidified part of the coating layer 3 contains a solidified material derived from a silane coupling agent. The sea-like solidified part containing the above-described solidified material securely combines with the island-shaped solidified part to form the coating layer 3 having a high strength. It is preferable that the silicone-based resin for use in the coating layer 3 consists of thermosetting silicone-based resin.

It is preferable that the composition containing the reactive silicone-based resin which is the main component of the island-shaped solidified part is thermosetting silicone-based resin or room temperature-curing silicone-based resin. It is especially preferable that the composition containing the reactive silicone-based resin is the thermosetting silicone-based resin from the standpoint of workability and the like.

As the reactive silicone, polydimethylsiloxane having a terminal silanol group is preferable. It is especially preferable that the reactive silicone has the silanol group at its both terminals. When polysiloxane silicone having the terminal silanol group is used as the reactive silicone, a condensate of this reactive silicone has siloxane bonds in its entire main chain.

As the reactive silicone having the terminal silanol group, the polysiloxane silicone having the silanol group at its both terminals is preferable. Such polysiloxane silicone includes polydimethylsiloxane, polydiphenylsiloxane, and diphenylsiloxane-dimethylsiloxane copolymer each having the silanol group at both terminals thereof. Although the form of the reactive silicone is not limited to a specific one, it is possible to use the above-described reactive silicone siloxane compounds or polysiloxanes, comprising a condensate thereof, which are dispersed, emulsified, and dissolved in an aqueous medium, a copolymer emulsion formed by copolymerizing an alkoxysilyl group-containing vinyl monomer with other vinyl monomers as necessary, and an emulsion formed by combining the polysiloxane with an organic polymer.

It is favorable that the resin composition forming the coating layer 3 contains a second silicone compound different from the reactive silicone-based resin having the silanol group or the siloxane bond. As the second silicone compound, alkylalkoxysilane, phenylalkoxysilane, alkylphenoxysilane, aminoalkylalkoxysilane, and glycidoxyalkylalkoxysilane are preferable.

It is also favorable that the composition forming the coating layer 3 contains the alkylalkoxysilane or the phenylalkoxysilane as the second silicone compound and the aminoalkylalkoxysilane or/and the glycidoxyalkylalkoxysilane as a third silicone compound.

It is more favorable that the resin composition forming the coating layer 3 contains the alkylalkoxysilane or the phenylalkoxysilane as the second silicone compound, the aminoalkylalkoxysilane as the third silicone compound, and the glycidoxyalkylalkoxysilane as a fourth silicone compound.

As the second silicone compound, the alkylalkoxysilane, the alkylphenoxysilane, and the phenylalkoxysilane are preferable. The alkylalkoxysilane has at least one alkyl group having 1 to 20 carbon atoms and at least one alkoxy group having 1 to 4 carbon atoms. As the alkylalkoxysilane, methyltrimethoxysilane, methyltriethoxysilane, methyltriisobutoxysilane, methyltributoxysilane, methylsec-trioctyloxysilane, isobutyltrimethoxysilane, cyclohexylmethyldimethoxysilane, diisopropyldimethoxysilane, propyltrimethoxysilane, diisobutyldimethoxysilane, n-octylmethoxysiloxane, ethyltrimethoxysilane, dimethyldimethoxysilane, octamethylcyclotetrasiloxane, methyltri(acryloyloxyethoxy)silane, octyltriethoxysilane, lauryltriethoxysilane, stearyl trimethoxysilane, stearyl triethoxysilane, ethyltriethoxysilane, propyltriethoxysilane, butyltriethoxysilane, butyltrimethoxysilane, pentyltrimethoxysilane, pentyltriethoxysilane, heptyltrimethoxysilane, heptyltriethoxysilane, octyltrimethoxysilane, nonyltrimethoxysilane, nonyltriethoxysilane, decyltrimethoxysilane, decyltriethoxysilane, undecyltrimethoxysilane, undecyltriethoxysilane, dodecyltrimethoxysilane, dodecyltriethoxysilane, tridecyltrimethoxysilane, tridecyltriethoxysilane, tetradecyltrimethoxysilane, tetradecyltriethoxysilane, pentadecyltrimethoxysilane, pentadecyltriethoxysilane, hexadecyltrimethoxysilane, hexadecyltriethoxysilane, heptadecyltrimethoxysilane, heptadecyltriethoxysilane, octadecyltrimethoxysilane, octadecyltriethoxysilane, nonadecyltrimethoxysilane, eykosiltrimethoxysilane, and eykosiltriethoxysilane are preferable.

As the alkylphenoxysilane, methyltriphenoxysilane is preferable. As the phenoxyalkoxysilane, phenyltrimethoxysilane, phenyltriethoxysilane, diphenyldimethoxysilane, and diphenyldiethoxysilane are preferable.

As the second silicone compound, it is also possible to use methyltri(glycidyloxy)silane, trimethylchlorosilane, dimethyl chlorosilane, methyltrichlorosilane, tetraethoxysilane, heptadecafluorodecyl trimethoxysilane, tridecafluorooctyl trimethoxysilane, and tetra propoxy silane.

As the second silicone compound, the aminoalkylalkoxysilane may be used. As the aminoalkylalkoxysilane, 3-aminopropyltriethoxysilane, 3-(2-aminoethyl)aminopropyltrimethoxysilane, 3-(2-aminoethyl)aminopropylmethyldimethoxysilane, 3-aminopropyltrimethoxysilane, and 3-phenylaminopropyltrimethoxysilane are preferable.

As the second silicone compound, the glycidoxyalkylalkoxysilane may be used. As the glycidoxyalkylalkoxysilane, 3-glycidoxypropyltrimethoxysilane, 3-glycidoxypropyltriethoxysilane, 3-glycidoxypropylmethyldiethoxysilane, 3-glycidoxypropylmethyldimetoxysilane, 2-(3,4-epoxycyclohexyl)ethyltrimethoxysilane are preferable.

As the second silicone compound, it is possible to use silane compounds such as 3-ureidopropyltriethoxysilane, diallyl dimethyl silane, n-octyldimethylchlorosilane, tetraethoxysilane, and trifluoropropyl trimethoxysilane.

The composition forming the coating layer 3 may contain the second and third silicone compounds. It is preferable to select the second silicone compound from among the alkylalkoxysilane, the alkylphenoxysilane, and the phenylalkoxysilane. As the third silicone compound, it is preferable to use the aminoalkylalkoxysilane or the glycidoxyalkylalkoxysilane. The composition forming the coating layer 3 may contain the second, third, and fourth silicone compounds. It is preferable to select the second silicone compound from among the alkylalkoxysilane, the alkylphenoxysilane, and the phenylalkoxysilane. As the third silicone compound, the aminoalkylalkoxysilane is preferable. As the fourth silicone compound, glycidoxyalkylalkoxysilane is preferable.

Because the gasket 1 of the present invention has the above-described coating layer 3, the gasket has a stable sliding contact performance without applying a lubricant to the sliding contact surface thereof and is capable of maintaining sealing performance inside the medicine accommodation space. It is preferable that the coating layer 3 allows the gasket 1 to have an initial sliding contact resistance value not more than the maximum value of a dynamic sliding contact resistance value thereof. In other words, it is preferable that the initial sliding contact resistance value of the gasket having the coating layer is not more than the maximum value of the dynamic sliding contact resistance value thereof. The gasket satisfying the above-described condition is capable of starting favorable initial sliding contact and does not make an excessive initial movement.

As the aqueous silicone-based resin, it is possible to preferably use a polysiloxane complex aqueous emulsion having a core part made of a crosslinked polymer and a shell part, made of a non-crosslinked polymer, which covers the core part and having polysiloxane disposed in the vicinity of the surface of the shell part.

The method of forming the coating layer 3 is described below. In the method of forming the coating layer, a coating solution in which a required amount of the above-described silicone-based resin and additives are dispersed and suspended in purified water is prepared. The coating layer is obtained by applying the coating solution to the clean surface of the gasket and thereafter hardening it. At this time, the coating solution can be applied to the surface of the gasket by carrying out a known method such as a dip coating method, a spraying method, and the like. It is preferable to spray the coating solution to the surface of the object to be coated with the object being rotated (specifically, at 100 to 600 rpm). In applying the coating solution to the surface of the gasket by spraying it, it is preferable to do so after heating the portion of the gasket to be coated to 60 to 120 degrees C. Thereby the coating solution can be rapidly fixed to the surface of the portion of the gasket to be coated without water repellence.

As the method of hardening the coating solution, it may be left at a normal temperature, but it is preferable to harden it by heating it The method of thermally hardening the coating solution is not limited to a specific method, provided that the base material of the gasket is not modified or deformed. It is possible to use conventional methods such as hot air drying and a drying oven using infrared rays or a method of using a drier to be operated under a reduced pressure. The thickness of the coating layer 3 to be formed is 1 to 30µm and preferably 3 to 10µm. Such a coating layer can be easily formed by appropriately controlling the concentration of a mixed solution, the dipping method, and the spraying method.

In preparing the coating solution containing the silicone-based resin, a catalyst for accelerating thermosetting may be used as an additive.

As the catalyst, acid, alkali, amine, organic salts of metals, titanate, and borate are used. Organic acid salts such as zinc octylate, iron octylate or organic acid salts of cobalt, tin, and lead are preferable.

As the organic acid salts of tin, it is possible to use bis(2-ethylhexanoate)tin, bis(neodecanoate)tin, di-n-butylbis(2-ethylhexylmalate)tin, di-n-butylbis(2,4-pentanedionate)tin, di-n-butylbutoxychloro tin, di-n-butyldiacetoxy tin, di-n-butyl tin dilaurate, dimethyl tin di neodecanoate, dimethyl hydroxy(oleate)tin, and tin dioctyl dilaurate.

In preparing the coating solution containing the silicone-based resin, additives such as a surface active agent, alcohol, and the like may be used to uniformly emulsify, suspend, and disperse the coating solution.

As the surface active agent, anion surface active agents are preferable. Any of the anion surface active agents can be used. It is possible to use aliphatic monocarboxylate, polyoxyethylene alkyl ether carboxylate, N-acyl sarcosinate, N-acyl glutamate, dialkyl sulfosuccinate, alkane sulfonate, alpha olefin sulfonate, straight chain alkylbenzene sulfonate, molecular chain alkylbenzene sulfonate, naphthalene sulfonate-formaldehyde condensate, alkylnaphthalene sulfonate, N-methyl-N-acyl taurine, alkyl sulfate, polyoxyethylene alkyl ether sulfate, fat and oil sulfuric acid ester salt, alkyl phosphate, and polyoxyethylene alkylphenyl ether sulfate.

Nonionic surface active agents may be used. Any of the nonionic surface active agents may be used. It is possible to use polyoxyethylene alkyl ether, polyoxyalkylene derivatives, polyoxyethylene alkyl phenyl ether, polyoxyethylene sorbitan fatty acid ester, fatty acid alkanolamide, glycerin fatty acid ester, sorbitan fatty acid ester, polyoxyethylene alkylamine, and alkylalkanolamide.

The syringe 10 of the present invention has the outer cylinder 11, the gasket 1 slidably accommodated inside the outer cylinder 11, and a plunger 17 which has been mounted or can be mounted on the gasket 1.

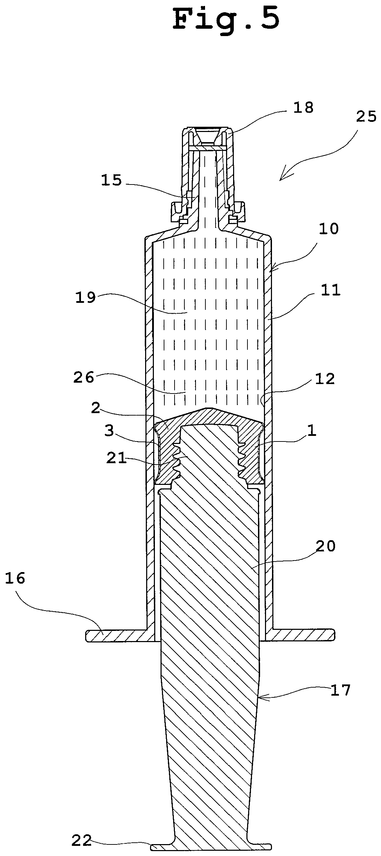

More specifically, as shown in Fig. 5, the syringe 10 is constructed of the outer cylinder 11, for use in the syringe, which has a needle-mounting portion 15 disposed at the distal side thereof and a flange 16 disposed at the proximal end thereof; the gasket 1, for use in the syringe, which is capable of making liquid-tight and airtight sliding contact with an inner surface 12 of the outer cylinder 11; the plunger 17 which has been or can be mounted on the gasket 1 for use in the syringe; a sealing member 18 for sealing the needle-mounting portion 15 of the outer cylinder 11 for use in the syringe; and a medicine-accommodating portion 19, for accommodating a medicine 26, which is formed among the sealing member 18, the inner surface 12 of the outer cylinder 11, and the gasket 1 for use in the syringe. Instead of the sealing member 18, a needle may be mounted on the needle-mounting portion 15.

As shown in Fig. 5, the sealing member 18 may be of a type having a piercing portion into which a double ended needle can be directly inserted or may be of a type in which a medicine cannot be discharged until the sealing member is removed. The gasket 1 has the coating layer 3. In the syringe 10, it is preferable that the dynamic sliding contact resistance value of the gasket 1 when the gasket 1 slides at a low speed (100mm/minute) inside the outer cylinder 11 is not more than 20N. Such a low dynamic sliding contact resistance value can be obtained in a case where the gasket 1 has the coating layer 3. It is especially preferable that the dynamic sliding contact resistance value of the gasket 1 when the gasket 1 slides at the low speed (100mm/minute) inside the outer cylinder 11 is 1N to 20N.

This medical appliance is a prefilled syringe 25 composed of the syringe 10 and the medicine 26, as shown in Fig. 5.

The outer cylinder 11 for use in the syringe is a cylindrical member having the needle-mounting portion 15 disposed at the distal portion thereof and the flange 16 disposed at the proximal end thereof. The outer cylinder 11 is made of a material transparent or semitransparent. It is preferable that the outer cylinder 11 is made of a material having a low oxygen permeability and a low vapor permeability. It is preferable that the material forming the outer cylinder 11 has a glass transition point or a melting point not less than 110 degrees C.

As the material forming the outer cylinder 11, various general-purpose rigid plastic materials are preferable. Polyolefin such as polypropylene, polyethylene, poly (4-methylpentene-1), and cyclic polyolefin; polyesters such as polyethylene terephthalate, polyethylene naphthalate, and non-crystalline polyarylate; polystyrene; polyamide; polycarbonate, polyvinyl chloride; acrylic resin; acrylonitrile-butadiene-styrene copolymer, and non-crystalline polyetherimide are preferable. The polypropylene, the poly (4-methylpentene-1), the cyclic polyolefin, the polyethylene naphthalate, and the non-crystalline polyetherimide are especially preferable because these resins are transparent and resistant to heat sterilization. These resins can be used as materials to form not only a syringe barrel, but also a container capable of accommodating a medicine. It is also possible to use glass as a material to form the outer cylinder.

As shown in Fig. 5, the plunger 17 has a sectionally cross-shaped main body 20 extended axially; a plunger-side threaded engaging portion 21, provided at the distal portion of the plunger 17, which threadedly engages the plunger-mounting portion 4; a disk-shaped gasket-pressing portion provided between the plunger-side threaded engaging portion 21 and the main body 20; a disk portion 22, for pressing use, which is disposed at the proximal end of the main body 20; and a disk-shaped rib formed midway on the main body 20.

The medicine 26 is contained inside the syringe 10 of this embodiment. As the medicine 26, it is possible to use a solution and a solid agent such as a powdery medicine and a freeze-dried medicine. When a liquid medicine having a poor water solubility and a high adsorbability or a liquid medicine containing a surface active agent and having a low viscosity and a high degree of penetration is accommodated inside the syringe 10, it is unnecessary to use silicone oil. When the coating layer 3 is provided at a portion which contacts an accommodated medicine, the adsorption of the medicine can be prevented. Thus it is possible to preferably use the syringe 10 for the liquid medicine having the above-described properties.

As the material for composing the plunger 17 and the sealing member 18, it is preferable to use hard resin or semi-hard resin such as polyvinyl chloride, high-density polyethylene, polypropylene, polystyrene, polyethylene terephthalate, polycarbonate, acrylic resin, and the like.

### EXAMPLES

Examples of the present invention are described below.

### Example 1

By using butyl rubber, the core part of a gasket, for use in a syringe, having a configuration shown in Figs. 1 and 2 was produced. The core part was formed by press-molding a vulcanizable rubber composition composed of butyl rubber and an additive added thereto. Describing the configuration of the obtained core part, it had a length of 20mm, an outer diameter of 23.7mm at distal-side and proximal-side annular ribs, a length of 10mm between the center of the distal-side annular rib and the center of the proximal-side annular rib, an outer diameter of 21.5mm at an identical diameter portion between the distal-side annular rib and the proximal-side annular rib, a length (depth) of 8mm in the plunger-mounting concave portion having a female screw at an inner side thereof, an inner diameter of 14.5mm at the distal side of the plunger-mounting concave portion, and an inner diameter of 15mm at the rear side of the plunger-mounting concave portion.

Thereafter 29 parts by weight of silicone-based resin and 1 part by weight of tin dioctyl dilaurate were added to 66 parts by weight of purified water to prepare a coating solution. The following silicone-based resins shown below were mixed with one another by using straight-chain sodium alkylbenzene sulfonate.

1) 25 parts by weight of 1501 Fluid ((commercial name), produced by Toray Dow Corning Corporation) containing polydimethylsiloxane having a silanol group at its both terminals as its main component
2) 0.1 parts by weight of Z-6366 ((commercial name), produced by Toray Dow Corning Corporation) containing methyltrimethoxysilane as its main component
3) 1 part by weight (content ratio of reaction product 50%) of an ethanol solution which was a reaction product of Z-6011 ((commercial name), produced by Toray Dow Corning Corporation) containing 3-aminopropyltriethoxysilane as its main component and maleic anhydride
4) 0.5 parts by weight of Z-6040 ((commercial name), produced by Toray Dow Corning Corporation) containing 3-glycidoxypropyltrimethoxysilane as its main component

After the gasket core member produced in the above-described manner was heated to 90 degrees C for 30 minutes at a room temperature and a normal pressure, the gasket core member was rotated (300 rpm) on its axis with the coating solution having the above-described composition being sprayed to the rotating gasket core member from the side surface thereof. Thereafter the coating solution was dried at 150 degrees C for 30 minutes. Thereby the gasket of the present invention was produced. Thereafter to wash extra coating solution which remained on the produced gasket, cleaning was performed with purified water having a temperature not less than 80 degrees C. The average thickness of a coating layer 3 formed on the surface of the core member was about 8µm. This gasket was set as the example 1.

### Example 2

As in the case of the example 1, 29 parts by weight of the silicone-based resin and 1 part by weight of the tin dioctyl dilaurate were added to 66 parts by weight of the purified water to prepare a main agent.

5 parts by weight of purified water was added to 8 parts by weight of the main agent and both were mixed with each other to prepare a coating solution. After at a room temperature and a normal pressure, the gasket core member produced in the above-described manner was heated to 90 degrees C for 30 minutes, the gasket core member was rotated (300 rpm) on its axis with the coating solution having the above-described composition being sprayed to the rotating gasket core member from the side surface thereof. Thereafter the coating solution was dried at 150 degrees C for 30 minutes. Thereby the gasket of the present invention was produced. Thereafter to wash extra coating solution which remained on the produced gasket, cleaning was performed with purified water having a temperature not less than 80 degrees C. The average thickness of a coating layer 3 formed on the surface of the core member was about 5µm. This gasket was set as the example 2.

### Example 3

29 parts by weight of the silicone-based resin and 1 part by weight of the tin dioctyl dilaurate were added to 66 parts by weight of the purified water to prepare a coating solution. As the silicone-based resin, substances shown below were mixed with one another by using the straight-chain sodium alkylbenzene sulfonate.
1) 25 parts by weight of DMS-S14 ((commercial name), produced by GELEST Inc.) containing the polydimethylsiloxane having the silanol group at its both terminals as its main component
2) 0.1 parts by weight of SIP6560.0 ((commercial name), produced by GELEST Inc.) containing the methyltrimethoxysilane as its main component
3) 1 part by weight (content ratio of reaction product 50%) of the ethanol solution which was a reaction product of SIA0610.0 ((commercial name), produced by GELEST Inc.) containing the 3-aminopropyltriethoxysilane as its main component and the maleic anhydride
4) 0.5 parts by weight of SIG5840.1 ((commercial name), produced by GELEST Inc.) containing the 3-glycidoxypropyltrimethoxysilane as its main component

After the gasket core member produced in the above-described manner was heated to 90 degrees C for 30 minutes at a room temperature and a normal pressure, the gasket core member was rotated (300 rpm) on its axis with the coating solution having the above-described composition being sprayed to the rotating gasket core member from the side surface thereof. Thereafter the coating solution was dried at 150 degrees C for 30 minutes. Thereby the gasket of the present invention was produced. Thereafter to wash extra coating solution which remained on the produced gasket, cleaning was performed with purified water having a temperature not less than 80 degrees C. The average thickness of a coating layer formed on the surface of the core member was about 8µm. This gasket was the as the example 3.

### Example 4

29 parts by weight of the silicone-based resin and 1 part by weight of the tin dioctyl dilaurate were added to 66 parts by weight of the purified water to prepare a coating solution. As the silicone-based resin, substances shown below were mixed with one another by using the straight-chain sodium alkylbenzene sulfonate.
1) 25 parts by weight of YR3204 ((commercial name), produced by Momentive Performance Materials Japan, Limited Liability Company) containing poly alkyl phenyl siloxane having a silanol group as its main component
2) 0.1 parts by weight of TSL8178 ((commercial name), produced by Momentive Performance Materials Japan, Limited Liability Company) containing phenyltriethoxysilane as its main component
3) 1 part by weight (content ratio of reaction product 50%) of the ethanol solution which was a reaction product of TSL8331 ((commercial name), produced by Momentive Performance Materials Japan, Limited Liability Company) containing the 3-aminopropyltriethoxysilane as its main component and the maleic anhydride
4) 0.5 parts by weight of TSL8350 ((commercial name), produced by Momentive Performance Materials Japan, Limited Liability Company) containing the 3-glycidoxypropyltrimethoxysilane as its main component

After the gasket core member produced in the above-described manner was heated to 90 degrees C for 30 minutes at a room temperature and a normal pressure, the gasket core member was rotated (300 rpm) on its axis with the coating solution having the above-described composition being sprayed to the rotating gasket core member from the side surface thereof. Thereafter the coating solution was dried at 150 degrees C for 30 minutes. Thereby the gasket of the present invention was produced. Thereafter to wash extra coating solution which remained on the produced gasket, the coating solution was washed with purified water having a temperature not less than 80 degrees C. The average thickness of a coating layer formed on the surface of the core member was about 8µm. This gasket was set as the example 4.

### Comparison Example 1

29 parts by weight of the silicone-based resin and 1 part by weight of the tin dioctyl dilaurate were added to 66 parts by weight of the purified water to prepare a coating solution. As the silicone-based resin, substances shown below were mixed with one another by using the straight-chain sodium alkylbenzene sulfonate
1) 25 parts by weight of 1501 Fluid ((commercial name), produced by Toray Dow Corning Corporation) containing the polydimethylsiloxane having a silanol group at its both terminals as its main component
2) 4 parts by weight of 360 Medical Fluid 12500((commercial name), produced by Toray Dow Corning Corporation) comprising polydimethylsiloxane having the methyl group at its both terminals

After the gasket core member produced in the above-described manner was heated to 90 degrees C for 30 minutes at a room temperature and a normal pressure, the gasket core member was rotated (300 rpm) on its axis with the coating solution having the above-described composition being sprayed to the rotating gasket core member from the side surface thereof. Thereafter the coating solution was dried at 150 degrees C for 30 minutes. Thereby the gasket was produced. Thereafter to wash extra coating solution which remained on the produced gasket, cleaning was performed with purified water having a temperature not less than 80°C. The average thickness of a coating layer formed on the surface of the core member was about 6µm. This gasket was set as the comparison example 1.

### Comparison Example 2

360 Medical Fluid 12500((commercial name), produced by Toray Dow Corning Corporation)) comprising polydimethylsiloxane having methyl groups at its both terminals was used as a coating solution. After a core member of a gasket produced in the above-described manner was subjected to heat treatment at 90 degrees C for 30 minutes at a room temperature and a normal pressure, the core member was impregnated with the coating solution having the above-described composition. Thereafter the coating solution was dried at 150 degrees C for 30 minutes to homogenize the coating solution. In this way, a gasket was produced. The coating layer formed on the surface of the core member was oily. The average thickness of the coating layer was approximately 1µm. The gasket was set as a comparison example 2.

### Experiment 1: Friction Coefficient Measurement Test

The coating solutions prepared in the examples 1 through 4 and the comparison examples 1 and 2 were applied with a brush respectively to EPDM rubber sheets, having a size of 30mm × 50mm and a thickness of 2mm, which was heated to 90 degrees C for 30 minutes. After the coating solutions were dried at 150 degrees C for 30 minutes, the rubber sheets were allowed to stand for 24 hours at a room temperature. A stainless steel ball (diameter: 10mm) was placed on the surface of each rubber sheet. A stress generated when the stainless steel ball was horizontally moved a distance of 20mm at a speed of 3mm/second was measured by using a surface nature measuring device (Type:22 produced by Shinto Scientific Co., Ltd.) to determine a dynamic friction coefficient (µd). Table 1 shows the results.

**Table 1**

| | µd |
|---|---|
| Example 1 | 0.12 |
| Example 2 | 0.11 |
| Example 3 | 0.13 |
| Example 4 | 0.12 |
| Comparison example 1 | 0.12 |
| Comparison example 2 | 0.10 |

### Checking on Cross-Sectional Property

The gasket of the example 1 was cut. Thereafter an enlarged image of a section of the coating layer 3 prepared by using a frozen ultra-thin sections method was photographed by using the transmission electron microscope (H-7100 type, produced by Hitachi, Ltd., accelerating voltage: 100kV). The image of the coating layer 3 observed in an axial section of the gasket is as shown in Fig. 6. The image of the coating layer 3 in a section vertical to the axial section of the gasket is as shown in Fig. 7.

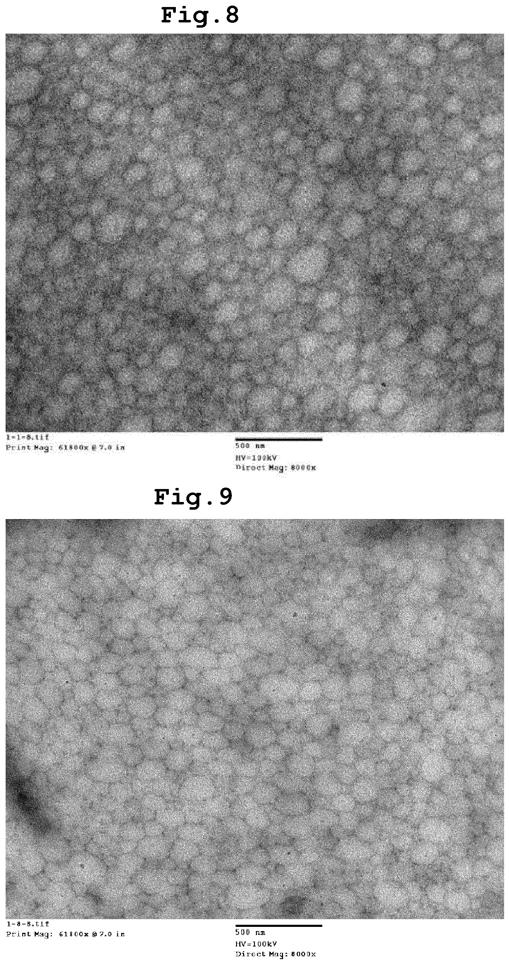

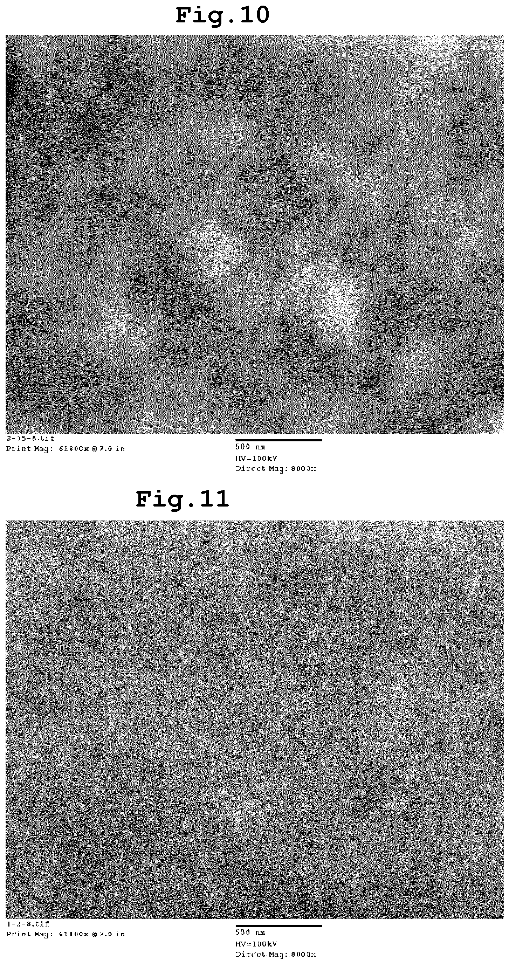

The image of the coating layer 3 in an axial section of the gasket of the example 2 is as shown in Fig. 8. The image of the coating layer 3 in an axial section of the gasket of the example 3 is as shown in Fig. 9. The image of the coating layer 3 in an axial section of the gasket of the example 4 is as shown in Fig.10.

The image of the coating layer 3 in an axial section of the gasket of the comparison example 1 is as shown in Fig. 11. In an attempt of observing a portion corresponding to the coating layer 3 which is the elastic solidified material layer in an axial section of the gasket of the comparison example 2, sea-like solidified parts were not observed.

From the images of the coating layers 3 of the examples 1 through 4 and the comparison example 1, the size (diameter) of one island-shaped solidified part of each coating layer and area occupancy ratios of the island-shaped solidified parts calculated by executing image processing (binarization processing) are as shown in table 2 below.

**Table 2**

| | Diameter (µm) | Area (µm²) | Area ratio (%) |
|---|---|---|---|
| Example 1 | 0.1∼0.6 | 0.008∼0.3 | 89 |
| Example 2 | 0.05∼0.3 | 0.002∼0.07 | 93 |
| Example 3 | 0.1∼0.2 | 0.008∼0.03 | 91 |
| Example 4 | 0.2∼0.5 | 0.03∼0.2 | 84 |
| Comparison example 1 | 0.2∼0.4 | 0.03∼0.13 | 70 |

### Experiment 2: Sliding Contact Resistance Measurement Test

As a material for outer cylinders for syringes, polypropylene (produced by Japan Polychem Corporation) was injection-molded to produce outer cylinders for syringes having the configuration shown in Fig. 5. The cylindrical portion of each outer cylinder for use in the syringe had an inner diameter of 23.5mm and a length of 95mm. As a material for plungers, the polypropylene (produced by Nippon Polychem Co., Ltd.) was injection-molded to form plungers having the configuration shown in Fig. 5.

The outer cylinders for the syringes, the gaskets of the examples 1 through 4 and the comparison examples 1 and 2, and the plungers were assembled to form syringes.

The sliding contact resistance value of each syringe was measured by an autograph (model name: EZ-Test, manufactured by Shimazu Seisakusho Co., Ltd.). More specifically, with the distal end of each syringe and the proximal end of each plunger being fixed to a fixing portion of the autograph to which an object to be measured is fixed, an initial sliding contact resistance value of each syringe and a maximum sliding contact resistance value (N) thereof were measured by moving the plungers downward 60mm at a speed of 100mm/minute. Table 3 shows the results.

As shown in table 3, in the syringes using the gaskets of the examples 1 through 4 and the comparison examples 1 and 2, the initial sliding contact resistance value and maximum sliding contact resistance value were almost equal to each other. In addition, there was a small difference between the initial sliding contact resistance value and maximum sliding contact resistance value. Thus there is little fear that a liquid medicine was discharged from the syringes in an amount more than a predetermined amount when the plunger was started to be pressed. Therefore the syringes were capable of discharging the liquid medicine safely and accurately. Favorable results that the initial sliding contact resistance value and the maximum sliding contact resistance value were not more than 10N were obtained.

**Table 3**

| | Sliding contact resistance value (N) | |
|---|---|---|
| | Initial time | Maximum |
| Example 1 | 4.9 | 7.0 |
| Example 2 | 5.2 | 7.2 |
| Example 3 | 5.0 | 7.1 |
| Example 4 | 5.3 | 7.5 |
| Comparison example 1 | 6.6 | 7.8 |
| Comparison example 2 | 6.8 | 7.2 |

### Experiment 3: Interlaminar Fracture Test)

After the sliding contact resistance measurement test of the experiment 2 finished, the outside appearances of the outer cylinders for the syringe and those of the gaskets of the examples 1 through 4 and the comparison example 1 were observed to check whether delamination occurred.

Examples 1 through 4: delamination did not occur.

Comparison example 1: delamination occurred.

### INDUSTRIAL APPLICABILITY

The gasket for use in a syringe of the present invention is as described below.
(1) A gasket, for use in a syringe, which is liquid-tightly slidable inside an outer cylinder thereof, said gasket comprising a core part made of an elastic body and a coating layer formed on said core part at least a portion thereof which contacts said syringe; in a portion where said coating layer is formed, an outer surface of said core part is not exposed, and an outer surface layer of said core part is formed of said coating layer; said coating layer is an elastic solidified material layer made of a silicone-based resin composition; in an enlarged image of said coating layer observed in an axial section of said gasket by using a transmission electron microscope, said coating layer has island-shaped solidified parts and sea-like solidified parts each positioned between said island-shaped solidified parts and linking said island-shaped solidified parts to each other and does not substantially have pin holes; and said island-shaped solidified part is composed mainly of aggregates of reactive silicone.
   The coating layer made of the elastic solidified material layer has the island-shaped solidified parts and sea-like solidified parts each positioned between the island-shaped solidified parts and linking the island-shaped solidified parts to each other. Thereby delamination hardly occurs in the coating layer. Therefore when the gasket slides in contact with the outer cylinder, the coating layer is stable and maintains preferable sliding contact performance.
   The embodiment of the present invention may be as described below.
(2) A gasket for use in a syringe according to the above (1), wherein said enlarged image of said coating layer observed in said axial section of said gasket and in a section vertical to said axial section thereof by using said transmission electron microscope, said coating layer has said island-shaped solidified parts and said sea-like solidified parts each positioned between said island-shaped solidified parts and linking said island-shaped solidified parts to each other; and does not substantially have pin holes.
(3) A gasket for use in a syringe according to the above (1) or (2), wherein said coating layer has a thickness of 3 to 30µm.
(4) A gasket for use in a syringe according to any one of the above (1) through (3), wherein said island-shaped solidified part is approximately circular and has a diameter of 0.05 to 0.8µm.
(5) A gasket for use in a syringe according to any one of the above (1) through (3), wherein an area of each of said island-shaped solidified part is 0.002 to 0.5µm².
(6) A gasket for use in a syringe according to any one of the above (1) through (5), wherein an area occupancy ratio of said island-shaped solidified parts as observed in said section of said coating layer is 80 to 95%.
(7) A gasket for use in a syringe according to any one of the above (1) through (6), wherein said coating layer is a compressed solidified material formed due to drying.
(8) A gasket for use in a syringe according to any one of the above (1) through (7), wherein said island-shaped solidified part and said sea-like solidified part have different properties.
(9) A gasket for use in a syringe according to any one of the above (1) through (8), wherein said island-shaped solidified part has a higher elasticity than said sea-like solidified part.
(10) A gasket for use in a syringe according to any one of the above (1) through (9), wherein said sea-like solidified part contains a solidified material derived from a silane coupling agent.
(11) A gasket for use in a syringe according to any one of the above (1) through (10), wherein said silicone-based resin is thermosetting silicone-based resin.
(12) A gasket for use in a syringe according to any one of the above (1) through (11), wherein an outer cylinder made of plastic is used.
   The syringe of the present invention is as described below.
(13) A syringe comprising an outer cylinder, a gasket, according to any one of the above (1) through (11), which is slidably accommodated inside said outer cylinder, and a plunger which has been mounted on said gasket or can be mounted thereon.
   The embodiment of the present invention may be as described below.
(14) A syringe according to the above (13), wherein a liquid medicine is filled.
(15) A syringe according to the above (13) or (14), wherein said outer cylinder is made of plastics.

## Claims

1. A gasket, for use in a syringe, which is liquid-tightly slidable inside an outer cylinder thereof,
said gasket comprising a core part made of an elastic body and a coating layer formed on said core part at least a portion thereof which contacts said syringe; in a portion where said coating layer is formed, an outer surface of said core part is not exposed, and an outer surface layer of said core part is formed of said coating layer; said coating layer is an elastic solidified material layer made of a silicone-based resin composition; in an enlarged image of said coating layer observed in an axial section of said gasket by using a transmission electron microscope, said coating layer has island-shaped solidified parts and sea-like solidified parts each positioned between said island-shaped solidified parts and linking said island-shaped solidified parts to each other and does not substantially have pin holes; and said island-shaped solidified part is composed mainly of aggregates of reactive silicone.

2. A gasket for use in a syringe according to claim 1, wherein said enlarged image of said coating layer observed in said axial section of said gasket and in a section vertical to said axial section thereof by using said transmission electron microscope, said coating layer has said island-shaped solidified parts and said sea-like solidified parts each positioned between said island-shaped solidified parts and linking said island-shaped solidified parts to each other; and does not substantially have pin holes.

3. A gasket for use in a syringe according to claim 1 or 2, wherein said coating layer has a thickness of 3 to 30µm.

4. A gasket for use in a syringe according to any one of claims 1 through 3, wherein said island-shaped solidified part is approximately circular and has a diameter of 0.05 to 0.8µm.

5. A gasket for use in a syringe according to any one of claims 1 through 3, wherein an area of each of said island-shaped solidified part is 0.002 to 0.5µm².

6. A gasket for use in a syringe according to any one of claims 1 through 5, wherein an area occupancy ratio of said island-shaped solidified parts as observed in said section of said coating layer is 80 to 95%.

7. A gasket for use in a syringe according to any one of claims 1 through 6, wherein said coating layer is a compressed solidified material formed due to drying.

8. A gasket for use in a syringe according to any one of claims 1 through 7, wherein said island-shaped solidified part and said sea-like solidified part have different properties.

9. A gasket for use in a syringe according to any one of claims 1 through 8, wherein said island-shaped solidified part has a higher elasticity than said sea-like solidified part.

10. A gasket for use in a syringe according to any one of claims 1 through 9, wherein said sea-like solidified part contains a solidified material derived from a silane coupling agent.

11. A gasket for use in a syringe according to any one of claims 1 through 10, wherein said silicone-based resin is thermosetting silicone-based resin.

12. A gasket for use in a syringe according to any one of claims 1 through 11, wherein an outer cylinder made of plastic is used.

13. A syringe comprising an outer cylinder, a gasket, according to any one of claims 1 through 11, which is slidably accommodated inside said outer cylinder, and a plunger which has been mounted on said gasket or can be mounted thereon.

14. A syringe according to claim 13, wherein a liquid medicine is filled.

15. A syringe according to claim 13 or 14, wherein said outer cylinder is made of plastics.
